# FASCICULE DE BREVET EUROPEEN

(11) **EP 2 509 570 B1**
(45) Date de publication et mention de la délivrance du brevet: **07.10.2015**
(21) Numéro de dépôt: 10799101.0
(22) Date de dépôt: 25.11.2010
(51) Int. Cl.: A61K 8/41, A61Q 17/00

(54) **UTILISATION DE DÉRIVÉS DE BENZYLOXY-ÉTHYLAMINES COMME CONSERVATEUR, PROCÉDÉ DE CONSERVATION ET COMPOSITIONS**
VERWENDUNG VON BENZYLOXY-ETHYLAMINDERIVATEN ALS KONSERVIERUNGSSTOFF, KONSERVIERUNGSVERFAHREN UND ZUSAMMENSETZUNGEN
USE OF BENZYLOXY-ETHYLAMINE DERIVATIVES AS A PRESERVATIVE, PRESERVATION METHOD, AND COMPOSITIONS

(30) Priorité: 11.12.2009 FR 0958867; 17.12.2009 US 287378 P
(43) Date de publication de la demande: 17.10.2012
(73) Titulaire: L'Oréal, 75008 Paris (FR)
(72) Inventeur: ROZOT, Roger, F-77400 Lagny/Marne (FR); DALKO, Maria, F-78000 Versailles (FR)
(74) Mandataire: Kromer, Christophe
(86) Numéro de dépôt international: PCT/FR2010/052520
(87) Numéro de publication internationale: WO 2011/070264

(56) Documents cités:
- EP-A1- 1 496 076
- WO-A1-01/94322
- anonymous: "2-(benzyloxy)-1-ethanamine hydrochloride", , 25 novembre 2005 (2005-11-25), XP002580055, Extrait de l'Internet: URL:www.maybridge.com [extrait le 2010-04-27]
- anonymous: "2-(benzyloxy)ethanamine", , 2006, XP002580056, Extrait de l'Internet: URL:www.chembridge.com [extrait le 2010-04-27]

## Description

La présente invention se rapporte à l'utilisation de composés de la famille des benzyloxy-éthylamines comme agents conservateurs, notamment dans des compositions cosmétiques, dermatologiques ou pharmaceutiques, et auxdites compositions.

Il est courant d'introduire dans les compositions cosmétiques ou dermatologiques, des conservateurs chimiques destinés à lutter contre le développement des microorganismes dans ces compositions, ce qui les rendrait rapidement inaptes à l'utilisation. Il faut notamment protéger les compositions contre les microorganismes susceptibles de se développer à l'intérieur de la composition et également contre ceux que l'utilisateur pourrait y introduire en la manipulant, en particulier lors de la préhension avec les doigts de produits en pot.
L'efficacité des conservateurs utilisés classiquement est variable et leur formulation peut poser des problèmes de formulation, tels qu'incompatibilité, voire déstabilisation notamment des émulsions. Ils peuvent, par ailleurs, être la cause d'effets secondaires indésirables (irritation, allergie) en particulier sur les peaux sensibles. Ainsi, des conservateurs chimiques couramment utilisés sont notamment les parabènes et les composés libérateurs de formol; mais ces conservateurs présentent toutefois l'inconvénient de causer des irritations, en particulier sur les peaux sensibles, lorsqu'ils sont présents à des taux relativement importants. D'autres conservateurs connus sont les hydroxyacides organiques; mais ils peuvent également générer des irritations du fait de leur effet desquamant sur la peau, ce qui n'est pas toujours bien toléré.
Par ailleurs, le consommateur, soucieux de la préservation de l'environnement, recherche de plus en plus des conservateurs non écotoxiques.

Il subsiste donc le besoin d'agents conservateurs, notamment d'agents antimicrobiens, ayant une action au moins aussi efficace que les composés de l'art antérieur, mais n'en présentant pas les inconvénients.
La présente invention a pour but de proposer de nouveaux agents conservateurs, présentant notamment un spectre antimicrobien large, et ne présentant pas les inconvénients de l'art antérieur.

Ainsi, un objet de la présente invention consiste en l'utilisation d'au moins un composé de formule (I) telle que définie ci-après, ou d'un de ses sels, comme agent conservateur.
Par agent conservateur, on entend une substance qui est ajoutée communément à une composition afin d'en assurer sa conservation vis-à-vis d'un agent contaminant. Avantageusement, les composés de formule (I) selon l'invention sont utilisés comme agent antimicrobien et/ou antibactérien et/ou antifongique.

Un autre objet de l'invention est un procédé de conservation d'une composition cosmétique, dermatologique ou pharmaceutique, caractérisé en ce qu'il consiste à incorporer à ladite composition au moins un composé de formule (I) ou l'un de ses sels, tel que défini ci-après.

Un autre objet de l'invention est une composition cosmétique, dermatologique ou pharmaceutique comprenant, dans un milieu physiologiquement acceptable, au moins un composé de formule (I), ou l'un de ses sels.

La Demanderesse a constaté de manière surprenante et inattendue que les composés de la famille des benzyloxy-éthylamines de formule (I) présentaient de bonnes propriétés antimicrobiennes, que cela soit vis-à-vis des bactéries, des levures ou des moisissures.
De par leur spectre antimicrobien large, ces composés peuvent donc être employés, notamment dans les compositions cosmétiques, comme agents antimicrobiens, notamment comme agents antibactériens, et/ou comme agents antifongiques c'est-à-dire comme agents anti-levures et/ou anti-moisissures. Ces composés de formule (I) peuvent donc être employés de manière avantageuse dans des compositions cosmétiques et/ou dermatologiques, notamment en tant qu'agent conservateur.
A la connaissance de la Demanderesse, les benzyloxy-éthylamines de formule (I) selon l'invention n'ont jamais été proposés dans des compositions cosmétiques, encore moins en tant qu'agent conservateur.

Les documents « 2-(benzyloxy)-1-ethanamine hydrochloride » 25.11.2005 extrait de l'internet URL : www.maybridge.com et «2-(benzyloxy)ethanamine » 2006, extrait de l'Internet URL www.chembridge.com sont des fiches produit de ces composés. Ces composés ne sont pas décrits comme conservateurs, ni formulés dans une composition cosmétique à une concentration comprise entre 0,1 à 5% en poids.

Les composés de formule (I) selon l'invention présentent comme avantage, une structure chimique clairement définie et caractérisée, d'où une reproductibilité de leur fabrication aisée; leur faisabilité industrielle est également assez simple.
De plus, ils possèdent une bonne solubilité ou compatibilité avec les milieux couramment employés en cosmétique, les milieux aqueux notamment.

Les composés selon l'invention répondent donc à la formule (I) suivante: 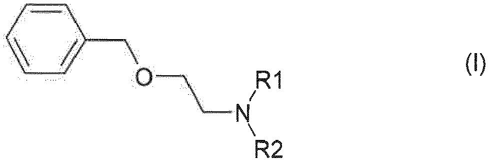 dans laquelle R1 et R2 représentent, indépendamment l'un de l'autre, un atome d'hydrogène ou un radical méthyle, éthyle, phényle ou benzyle;
ainsi que leurs sels.

De préférence, R1 représente H.

De préférence, R2 représente H ou benzyle.

Les sels, organiques ou minéraux, des composés de formule (I), font également partie de l'invention. Les composés salifiés répondent donc à la formule (I') : 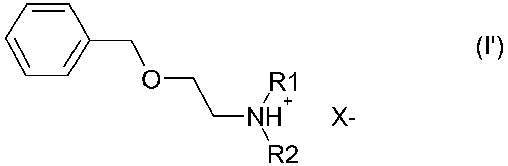 dans laquelle R1 et R2 sont tels que définis dans la formule (I) et X- est un anion, ou un mélange d'anions, organique et/ou minéral, cosmétiquement acceptable.

En particulier X- peut représenter un anion, ou un mélange d'anions, choisis parmi les halogénures, notamment chlorure, bromure, fluorure, iodure; un hydroxyde; un phosphate; un sulfate; un hydrogénosulfate; les alkylsulfates dans lesquels l'alkyle est linéaire ou ramifié, en C1-C6, comme méthylsulfate ou éthylsulfate; les carbonates et hydrogénocarbonates; les sels d'acides carboxyliques tels que le formiate, l'acétate, le citrate, le tartrate, l'oxalate; les alkylsulfonates dans lesquels l'alkyle est linéaire ou ramifié, en C1-C6, comme méthylsulfonate; les arylsulfonates dans lesquels l'aryle, de préférence phényle, est éventuellement substitué par un ou plusieurs radicaux alkyle en C1-C4 tel que par exemple le 4-toluylsulfonate; les alkylsulfonyles tel que le mésylate; leurs mélanges.

Comme composés de formule (I) selon l'invention, on peut en particulier citer :

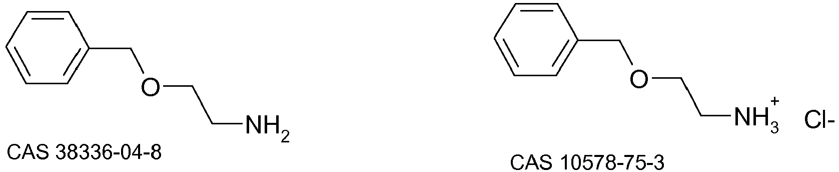

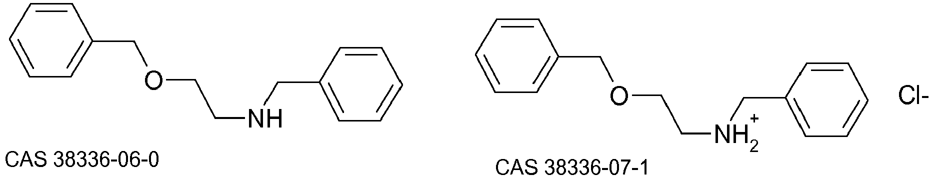

On peut bien évidemment utiliser un mélange de composés de formule (I).
De préférence, la composition ne comprend pas d'autres agents conservateurs que ceux de formule (I). En particulier, la composition ne contient pas de parabens.

Les composés de formule (I) sont, pour certains d'entre eux, disponibles commercialement, notamment auprès des sociétés Maybridge pour le composé de CAS 10578-75-3 ou Chembridge pour le composé de CAS 38336-04-8.
Ces composés peuvent être aisément synthétisés par l'homme du métier, par exemple selon les procédés suivants :
A/ les composés dans lesquels R1=R2=H peuvent être préparés en trois étapes à partir de l'éthanolamine via la formation d'un oxazoline intermédiaire. L'ouverture de ce dernier par l'alcool benzylique conduit à la formation du composé N-acétylé. La deprotection de groupe acétate peut être réalisée dans des conditions classiques, notamment avec H₃PO₄ à reflux ou dans des conditions basiques. 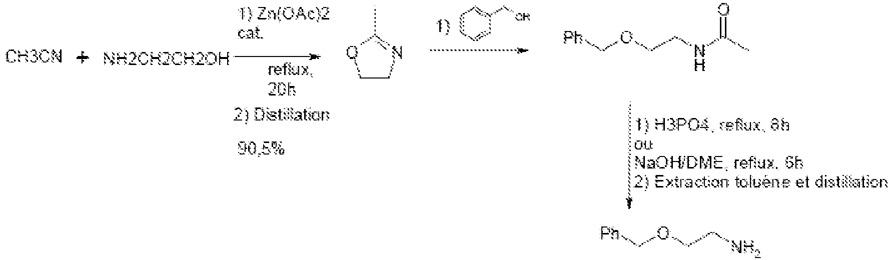
B/ les composés dans lesquels R1=H et R2 = phényle peuvent être préparés selon la synthèse décrite dans la littérature, notamment dans "Bioorganic & Med. Chem. Lett. 2006, 16(7) 1965-1968, par amination réductrice de l'aldéhyde de départ correspondant.

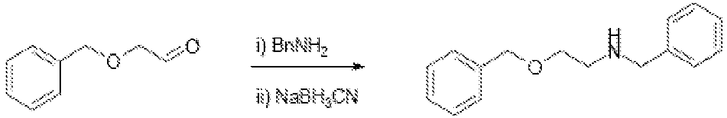

Les composés de la famille des benzyloxy-éthylamines de formule (I) selon l'invention peuvent être présents dans les compositions cosmétiques, dermatologiques ou pharmaceutiques en une quantité suffisante pour obtenir l'effet recherché, et représentent notamment 0,1 à 5% en poids, de préférence 0,25 à 3% en poids, et plus particulièrement 0,5 à 2% en poids, du poids total de la composition.
Les compositions comprenant les composés de formule (I) selon l'invention comprennent un milieu physiologiquement acceptable, c'est-à-dire compatible avec les matières kératiniques telles que la peau du visage ou du corps, les lèvres, les cheveux, les cils, les sourcils et les ongles.
Elles peuvent se présenter sous toutes les formes galéniques appropriées pour une application topique, notamment sous forme de solution aqueuse, hydroalcoolique, organique ou huileuse; de suspension ou de dispersion dans des solvants ou des corps gras, de type lotion ou sérum; sous forme de dispersion vésiculaire; sous forme d'émulsion E/H, H/E ou multiple telle qu'une crème ou un lait; sous forme de pommade, de gel, de bâtonnet solide, de produits anhydres pâteux ou solides, de mousse notamment aérosol ou de spray.

Le milieu physiologiquement acceptable dans lequel les composés peuvent être employés, ainsi que ses constituants, leur quantité, la forme galénique de la composition et son mode de préparation, peuvent être choisis par l'homme du métier sur la base de ses connaissances générales en fonction du type de composition recherchée.

Notamment, la composition peut comprendre tout corps gras usuellement utilisé dans le domaine d'application envisagé. On peut notamment citer les corps gras siliconés tels que les huiles, les gommes et les cires de silicone, ainsi que les corps gras non siliconés tels que les huiles, les pâteux et les cires d'origine végétale, minérale, animale et/ou synthétique. Les huiles peuvent éventuellement être volatiles ou non volatiles.
On peut encore citer les hydrocarbures, notamment les isoparaffines; les esters et les éthers de synthèse comprenant au total 8 à 60 atomes de carbone; les alcools gras en C8-C32 et les acides gras en C8-C32.
La composition peut également comprendre un milieu aqueux, un milieu hydroalcoolique contenant un monoalcool en C2-C6, tel que l'éthanol ou l'isopropanol, ou un milieu organique comprenant des solvants organiques usuels tels que des alcools en C2-C6, notamment l'éthanol et l'isopropanol, des glycols tels que le propylène glycol, des cétones.

La composition peut comprendre les adjuvants habituels dans le domaine considéré, tels que les émulsionnants, les tensioactifs, les épaississants ou gélifiants hydrophiles ou lipophiles, les actifs notamment cosmétiques, les antioxydants, les parfums, les charges, les pigments, les filtres UV, les absorbeurs d'odeur, les colorants, les hydratants (glycérine), les vitamines, les acides gras essentiels, les polymères, notamment liposolubles, les opacifiants, les stabilisants, les séquestrants, les conditionneurs, les agents propulseurs. Bien entendu, l'homme du métier veillera à choisir ce ou ces éventuels adjuvants et/ou leur quantité de manière telle que les propriétés avantageuses de la composition selon l'invention ne soient pas, ou substantiellement pas, altérées par l'adjonction envisagée.

Le pH des compositions selon l'invention, lorsqu'elles comprennent au moins une phase aqueuse (solutions aqueuses, émulsions par exemple), est de préférence compris entre 4 et 9, de préférence entre 4 et 7, avantageusement de 5 à 6, et en particulier un pH de 5,5.

La composition selon l'invention peut notamment se présenter sous la forme :
- d'un produit de maquillage de la peau du visage, du corps ou des lèvres, tel qu'un fond de teint, un fard à joues ou à paupières, un stick anticernes, un stick camouflant, un eye-liner, un mascara, un rouge à lèvres, un vernis-à-ongles, un soin des ongles;
- d'une composition de protection solaire ou de bronzage artificiel;
- d'un gel ou lotion après-rasage;
- d'une crème dépilatoire;
- d'une composition d'hygiène corporelle telle qu'un déodorant, un gel douche ou un shampooing;
- d'une composition pharmaceutique;
- d'une composition solide telle qu'un savon ou un pain de nettoyage;
- d'une composition pour aérosol comprenant également un agent propulseur sous pression;
- d'une lotion de mise en plis, d'une crème ou d'un gel coiffant, d'une composition de teinture (notamment d'oxydation), d'une lotion restructurante pour cheveux, d'une composition de permanente, d'une lotion ou d'un gel antichute;
- d'une composition à usage bucco-dentaire, par exemple une pâte dentifrice.

L'invention est illustrée plus en détail dans les exemples de réalisation suivants.

### Exemple 1 : Détermination de l'activité antimicrobienne de composés selon l'invention dans l'eau

L'efficacité antimicrobienne de composés de formule (I) a été évaluée par la méthode du Challenge Test ou contamination artificielle après avoir introduit lesdits composés dans de l'eau.

### Composés testés

| | Structure | Source |
|---|---|---|
| Composé A | 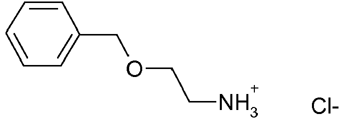 | MO 07103 de la société Maybridge |
| Composé B | 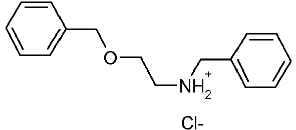 | Préparé à partir du composé non salifié * |

| | | |
|---|---|---|
| * Synthèse du composé B : le composé non salifié (CAS 38336-06-0) est préparé selon la synthèse décrite dans Bioorganic&Med.Chem.Letters (2006), 16(7), 1965-1968; ce composé est ensuite mélangé avec HCl (5N) dans l'isopropanol, et le composé B est récupéré par filtration, avec un rendement de 95%. | | |

### Protocole

La méthode du challenge-test consiste en une contamination artificielle de l'échantillon par des souches microbiennes de collection (bactéries, levures et moisissures) et en une évaluation du nombre de micro-organismes revivifiables sept jours après l'inoculation.

Afin de mettre en évidence l'effet d'un composé de formule (I), l'activité antimicrobienne d'une solution aqueuse comprenant une quantité "x" dudit composé a été comparée avec la même solution seule (témoin), après inoculation d'environ 10⁶ UFC (Unités formant des colonies)/gramme de solution aqueuse.

### Cultures de microorganismes

5 cultures pures de microorganismes sont utilisées.

| GERMES | Milieu de repiquage | T° | ATCC |
|---|---|---|---|
| *Escherichia coli* (Ec) | Trypto-caséine soja | 35°C | 8739 |
| *Enterococcus faecalis* (Ef) | Trypto-caséine soja | 35°C | 33186 |
| *Pseudomonas aeruginosa* (Pa) | Trypto-caséine soja | 35°C | 19429 |
| *Candida albicans* (Ca) | Sabouraud | 35°C | 10231 |
| *Aspergillus niger* (An) | Malt | 35°C | 6275 |

| | | | |
|---|---|---|---|
| ATCC = American Type Culture Collection | | | |

Les souches de bactéries gram - (*Escherichia coli* et *Pseudomonas aeruginosa*), bactérie gram + (*Enterococcus faecalis*), levure (*Candida albicans*), et moisissure (*Aspergillus niger*) sont ensemencées dans du milieu de repiquage, respectivement la veille de l'inoculation pour les bactéries et la levure, et 5 jours avant l'inoculation pour la moisissure.

Le jour de l'inoculation :
- on prépare respectivement pour les bactéries et la levure, une suspension dans du diluant Tryptone sel, de manière à obtenir au spectrophotomètre une suspension de densité optique comprise entre 35% et 45% de lumière transmise à 544 nm;
- pour la moisissure, on prélève les spores en lavant l'agar avec 6 à 7 ml de solution de récolte et on récupère la suspension dans un flacon ou un tube stérile.

Après avoir homogénéisé la suspension microbienne, on introduit dans chaque pilulier 0,2 ml d'inoculum (les suspensions sont utilisées pures: entre 1×10⁸ et 3×10⁸ UFC par ml) et on homogénéise parfaitement à l'aide d'une spatule la suspension microbienne dans les 20 g de produit (= solution aqueuse contenant les composés de formule (I) aux concentrations indiquées).

Le taux de micro-organismes présents dans le produit correspond après homogénéisation à une concentration de 10⁶ germes par gramme de produit, soit l'inoculation à 1 % d'un inoculum à 10⁸ germes par ml.
Après 7 jours de temps de contact entre les germes et le produit à 22°C ± 2°C et à l'obscurité, on réalise des dilutions décimales et on dénombre le nombre de micro-organismes revivifiables restant dans le produit.

### Résultats

| | | | Nb d'UFC/ gramme de produit à T7 jours (taux d'inoculation à 10⁶ germes/g) | | | | |
|---|---|---|---|---|---|---|---|
| | Taux | pH final | *E.coli* | *P. aeruginosa* | *E. faecalis* | *C. albicans* | *A*. *niger* |
| Composé A | 1% | 7,5 | <200 | <200 | <200 | <200 | 3,6.10⁴ |
| Composé A | 0,75% | 7,4 | <200 | <200 | <200 | 5,8.10³ | 1,6.10⁵ |
| Composé A | 0,5% | 7,4 | <200 | <200 | <200 | 1,3.10⁶ | 6,2.10⁵ |
| Composé A | 0,25% | 6,9 | 2,8.10³ | 3,0.10³ | 1,2.10³ | 2,3.10⁶ | 1,0.10⁶ |
| Composé A | 0,1% | 7,2 | 1,9.10⁶ | 1,5.10⁵ | 6,9.10⁴ | 2,8.10⁶ | 1,0.10⁶ |
| Composé A | 0,05% | 7 | 6,2.10⁶ | 1,9.10⁶ | 3,3.10⁵ | 3,0.10⁶ | 1,3.10⁶ |
| Composé A | 0,01% | 7 | 7,4.10⁶ | 9,0.10⁶ | 4,1.10⁵ | 3,2.10⁶ | 1,4.10⁶ |
| Composé B | 1% | 6,6 | <200 | <200 | <200 | <200 | <200 |

| | | | | | | | |
|---|---|---|---|---|---|---|---|
| <200 UFC : seuil de sensibilité de la méthode | | | | | | | |

Cette étude montre que les composés de formule (I) selon l'invention présentent un spectre antimicrobien très large du fait de leur activité antibactérienne et fongique. Ce sont des conservateurs efficaces, en particulier dans les solutions aqueuses testées.

### Exemple 2

On prépare une lotion comprenant (% en poids) :
- composé A 1%
- glycérine 2%
- alcool éthylique 20%
- butanol oxyéthyléné (26OE) oxypropyléné (26OP), huile de ricin hydrogénée oxyéthylénée (40 OE) dans l'eau 1 %
- eau déminéralisée qsp 100%

### Exemple 3

On prépare un gel pour le visage comprenant (% en poids) :
- composé A 1%
- Polyacrylate de glycéryle (Norgel) 30%
- Polyacrylamide/C13-14 Isoparaffine/laureth-7 (Sepigel 305) 2%
- Huile de silicone 10%
- Eau qsp 100%

### Exemple 4

On prépare un gel traitant comprenant (% en poids) :
- composé B 1%
- gomme de xanthane 1 %
- glycérine 2%
- éthanol 20%
- mélange alcool butylique oxyéthyléné (26OE) oxypropyléné (26OP), huile de ricin hydrogénée oxyéthylénée (40OE) dans l'eau 1 %
- parfum q.s.
- eau déminéralisée qsp 100%

### Exemple 5

On prépare une crème nettoyante moussante comprenant (% en poids) :
- monostéarate d'éthylène glycol 2%
- composé B 0,5%
- silicate de magnésium et d'aluminium hydraté 1,7%
- hydroxypropylméthylcellulose 0,8%
- mélange de cocoyl isethionate de sodium et d'acides gras de coprah (Elfan AT 84 G de Akzo) 15%
- acide stéarique 1,25%
- lauroyl sarcosinate de sodium à 30 % dans l'eau 10%
- parfum q.s.
- eau déminéralisée qsp 100%

### Exemple 6

On prépare une crème de soin comprenant (% en poids) :
- tristéarate de sorbitane 1 %
- composé A 1,5%
- homopolymère carboxyvinylique réticulé 0,4%
- gomme de xanthane 0,5%
- copolymère diméthacrylate d'éthylèneglycol/méthacrylate de lauryle 1 %
- cyclopentadiméthylsiloxane 6%
- glycérine 3%
- émulsionnant 4%
- parfum q.s.
- eau déminéralisée qsp 100%

### Exemple 7

On prépare un stick camouflant comprenant (% en poids) :
- cires (Carnauba et Ozokérite) 14%
- fraction liquide de beurre de karité 4%
- oxydes de titane et de zinc 22%
- oxydes de fer 4%
- composé A 1%
- polydiméthylsiloxane / silice hydratée 0,1%
- alcool cétylique 1,4%
- isoparaffine qsp 100%

### Exemple 8

On prépare une crème teintée comprenant (% en poids) :
- lécithine hydrogénée 2,4%
- huile d'amandes d'abricot 6%
- copolymère diméthacrylate d'éthylèneglycol/méthacrylate de lauryle 1 %
- stérols de soja oxyéthylénés (5 OE) 1,6%
- composé B 1%
- oxydes de fer 0,9%
- oxyde de titane 5%
- polyacrylamide / C₁₃-C₁₄-Isoparaffine / Laureth-7 (Sepigel 305) 4%
- cyclopentadiméthylsiloxane 6%
- glycérine 6%
- propylène glycol 6%
- parfum q.s.
- eau déminéralisée qsp 100%

## Revendications

1. Utilisation d'au moins un composé de formule (I), ou d'un de ses sels, comme agent conservateur : 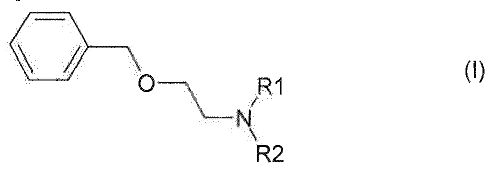 dans laquelle R1 et R2 représentent, indépendamment l'un de l'autre, un atome d'hydrogène ou un radical méthyle, éthyle, phényle ou benzyle.

2. Utilisation selon la revendication 1, comme agent conservateur d'une composition cosmétique, dermatologique ou pharmaceutique.

3. Utilisation selon l'une des revendications précédentes, dans laquelle R1 représente H.

4. Utilisation selon l'une des revendications précédentes, dans laquelle R2 représente H ou benzyle.

5. Utilisation selon l'une des revendications précédentes, dans laquelle les composés de formule (I) sont salifiés et répondent à la formule (I') : 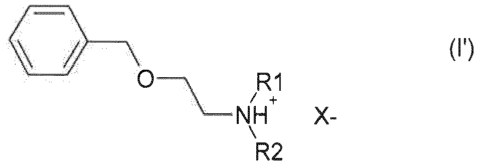 dans laquelle R1 et R2 sont tels que définis dans la formule (I) et X- est un anion, ou un mélange d'anions, organique et/ou minéral, cosmétiquement acceptable.

6. Utilisation selon l'une des revendications précédentes, dans laquelle le composé de formule (I) est choisi parmi :

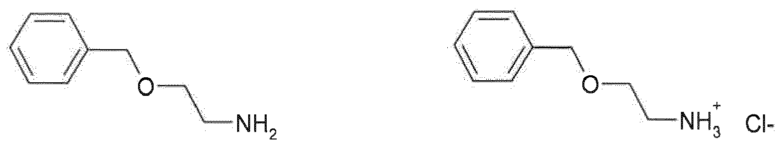

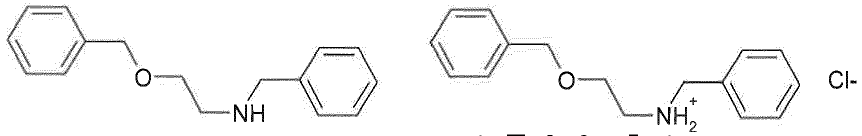

7. Utilisation selon l'une des revendications 2 - 6 dans laquelle le composé de formule (I) et/ou l'un de ses sels, seul ou en mélange, est présent dans les compositions cosmétiques, dermatologiques ou pharmaceutiques en une quantité comprise entre 0,1 à 5% en poids, de préférence 0,25 à 3% en poids, et plus particulièrement 0,5 à 2% en poids, du poids total de la composition.

8. Utilisation selon l'une des revendications 2 - 6 dans laquelle la composition se présente sous la forme :
- d'un produit de maquillage de la peau du visage, du corps ou des lèvres, tel qu'un fond de teint, un fard à joues ou à paupières, un stick anticernes, un stick camouflant, un eye-liner, un mascara, un rouge à lèvres, un vernis-à-ongles, un soin des ongles;
- d'une composition de protection solaire ou de bronzage artificiel;
- d'un gel ou lotion après-rasage;
- d'une crème dépilatoire;
- d'une composition d'hygiène corporelle telle qu'un déodorant, un gel douche ou un shampooing;
- d'une composition pharmaceutique;
- d'une composition solide telle qu'un savon ou un pain de nettoyage;
- d'une composition pour aérosol comprenant également un agent propulseur sous pression.;
- d'une lotion de mise en plis, d'une crème ou d'un gel coiffant, d'une composition de teinture, notamment d'oxydation; d'une lotion restructurante pour cheveux, d'une composition de permanente, d'une lotion ou d'un gel antichute;
- d'une composition à usage bucco-dentaire, par exemple une pâte dentifrice.

9. Procédé de conservation d'une composition cosmétique, dermatologique ou pharmaceutique, **caractérisé en ce qu'**il consiste à incorporer à ladite composition au moins un composé de formule (I) ou l'un de ses sels, tel que défini dans l'une quelconque des revendications 1 à 6.

10. Composition cosmétique, dermatologique ou pharmaceutique comprenant, dans un milieu physiologiquement acceptable, au moins un composé de formule (I), ou l'un de ses sels : 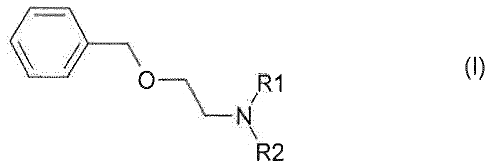 dans laquelle R1 et R2 représentent, indépendamment l'un de l'autre, un atome d'hydrogène ou un radical méthyle, éthyle, phényle ou benzyle;
le composé de formule (I) et/ou l'un de ses sels, seul ou en mélange, étant présent en une quantité comprise entre 0,1 à 5% en poids, du poids total de la composition.

11. Composition selon la revendication 10, dans laquelle les composés de formule (I) sont salifiés et répondent à la formule (I') : 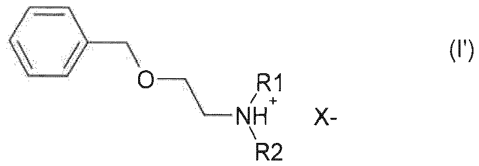 dans laquelle R1 et R2 représentent, indépendamment l'un de l'autre, un atome d'hydrogène ou un radical méthyle, éthyle, phényle ou benzyle; et X- est un anion, ou un mélange d'anions, organique et/ou minéral, cosmétiquement acceptable.

12. Composition selon l'une des revendications 10 à 11, dans laquelle le composé de formule (I) ou (I') est choisi parmi :

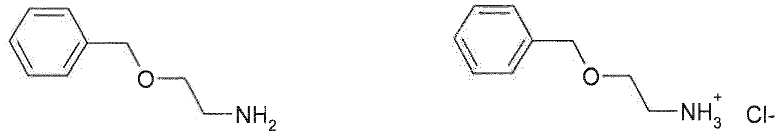

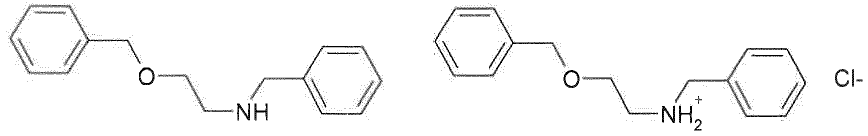

13. Composition selon l'une des revendications 10 à 12, dans laquelle le composé de formule (I) et/ou l'un de ses sels, seul ou en mélange, est présent en une quantité comprise entre 0,25 et 3% en poids, et plus particulièrement entre 0,5 et 2% en poids, du poids total de la composition.

14. Composition selon l'une des revendications 10 à 13, dans laquelle le milieu physiologiquement acceptable comprend au moins un ingrédient choisi parmi : les corps gras siliconés tels que les huiles, les gommes et les cires de silicone; les corps gras non siliconés tels que les huiles, les pâteux et les cires d'origine végétale, minérale, animale et/ou synthétique; un milieu aqueux; un milieu hydroalcoolique contenant un monoalcool en C2-C6; les alcools en C2-C6, les glycols tels que le propylène glycol; les cétones; les émulsionnants, les tensioactifs, les épaississants ou gélifiants hydrophiles ou lipophiles, les actifs notamment cosmétiques, les antioxydants, les parfums, les charges, les pigments, les filtres UV, les absorbeurs d'odeur, les colorants, les hydratants, les vitamines, les acides gras essentiels, les polymères notamment liposolubles, les opacifiants, les stabilisants, les séquestrants, les conditionneurs, les agents propulseurs.

15. Composition selon l'une des revendications 10 à 14, se présentant sous la forme :
- d'un produit de maquillage de la peau du visage, du corps ou des lèvres, tel qu'un fond de teint, un fard à joues ou à paupières, un stick anticernes, un stick camouflant, un eye-liner, un mascara, un rouge à lèvres, un vernis-à-ongles, un soin des ongles;
- d'une composition de protection solaire ou de bronzage artificiel;
- d'un gel ou lotion après-rasage;
- d'une crème dépilatoire;
- d'une composition d'hygiène corporelle telle qu'un déodorant, un gel douche ou un shampooing;
- d'une composition pharmaceutique;
- d'une composition solide telle qu'un savon ou un pain de nettoyage;
- d'une composition pour aérosol comprenant également un agent propulseur sous pression.;
- d'une lotion de mise en plis, d'une crème ou d'un gel coiffant, d'une composition de teinture, notamment d'oxydation; d'une lotion restructurante pour cheveux, d'une composition de permanente, d'une lotion ou d'un gel antichute;
- d'une composition à usage bucco-dentaire, par exemple une pâte dentifrice.

## Patentansprüche

1. Verwendung von mindestens einer Verbindung der Formel (I) oder eines ihrer Salze 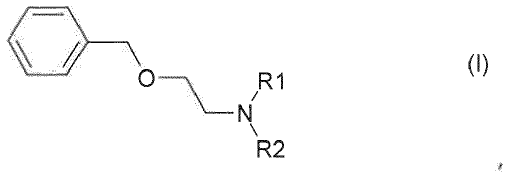 worin R1 und R2 unabhängig voneinander ein Wasserstoffatom oder einen Methyl-, Ethyl-, Phenyl- oder Benzylrest bedeuten,
als Konservierungsstoff.

2. Verwendung nach Anspruch 1 als Konservierungsstoff einer kosmetischen, dermatologischen oder pharmazeutischen Zusammensetzung.

3. Verwendung nach einem der vorhergehenden Ansprüche, wobei R1 H bedeutet.

4. Verwendung nach einem der vorhergehenden Ansprüche, wobei R2 H oder Benzyl bedeutet.

5. Verwendung nach einem der vorhergehenden Ansprüche, wobei die Zusammensetzungen der Formel (I) in ein Salz umgewandelt werden und der Formel (I') entsprechen: 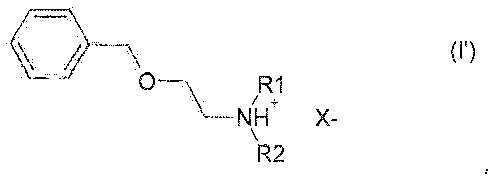 worin R1 und R2 wie in Formel (I) definiert sind und X- ein kosmetisch unbedenkliches organisches und/oder anorganisches Anion oder eine Mischung von Anionen ist.

6. Verwendung nach einem der vorhergehenden Ansprüche, wobei die Verbindung der Formel (I) aus den Folgenden ausgewählt ist:

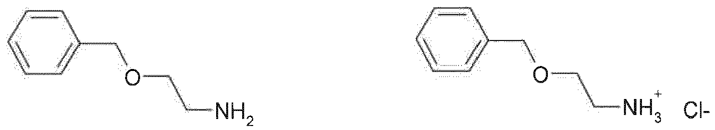

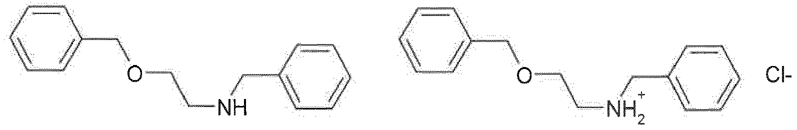

7. Verwendung nach einem der Ansprüche 2-6, wobei die Zusammensetzung der Formel (I) und/oder eines ihrer Salze, allein oder in Mischung, in den kosmetischen, dermatologischen oder pharmazeutischen Zusammensetzungen in einer Menge zwischen 0,1 bis 5 Gew.-%, vorzugsweise 0,25 bis 3 Gew.-% und stärker bevorzugt 0,5 bis 2 Gew.-% des Gesamtgewichts der Zusammensetzung vorliegt.

8. Verwendung nach einem der Ansprüche 2-6, wobei die Zusammensetzung in Form der Folgenden vorliegt:
- eines Make-up-Produkts der Haut des Gesichts, des Körpers oder der Lippen, wie einer Make-up-Grundlage, eines Rouge oder eines Lidschattens, eines Concealer-Sticks, eines Abdeck-Sticks, eines Eyeliners, einer Wimperntusche, eines Lippenstifts, eines Nagellacks, einer Nagelpflege;
- einer Sonnenschutzzusammensetzung oder einer Selbstbräunungszusammensetzung;
- eines Aftershave-Gels oder einer Aftershave-Lotion;
- einer Enthaarungscreme;
- einer Körperhygienezusammensetzung wie eines Deodorants, eines Duschgels oder eines Shampoos;
- einer pharmazeutischen Zusammensetzung;
- einer festen Zusammensetzung wie einer Seife oder eines Waschstücks;
- einer Zusammensetzung für Aerosol, die auch ein unter Druck stehendes Treibmittel umfasst;
- einer Lotion zum Wellenlegen, einer Frisiercreme oder eines Frisiergels, einer Färbezusammensetzung, insbesondere einer Oxidationszusammensetzung; einer restrukturierenden Lotion für das Haar, einer Dauerwellzusammensetzung, einer Lotion oder eines Gels gegen Haarausfall;
- einer Zusammensetzung für den Mund-Zahn-Bereich, zum Beispiel einer Zahncreme.

9. Verfahren zum Konservieren einer kosmetischen, dermatologischen oder pharmazeutischen Zusammensetzung, **dadurch gekennzeichnet, dass** es daraus besteht, in die Zusammensetzung mindestens eine Verbindung der Formel (I) oder eines ihrer Salze wie in einem der Ansprüche 1 bis 6 definiert einzuarbeiten.

10. Kosmetische, dermatologische oder pharmazeutische Zusammensetzung, die in einem physiologisch unbedenklichen Medium mindestens eine Verbindung der Formel (I) oder eines ihrer Salze 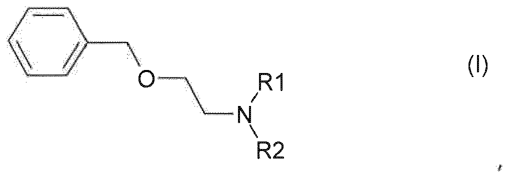 in der R1 und R2 unabhängig voneinander ein Wasserstoffatom oder einen Methyl-, Ethyl-, Phenyl- oder Benzylrest bedeuten,
umfasst, wobei die Verbindung der Formel (I) und/oder eines ihrer Salze, allein oder in Mischung, in einer Menge zwischen 0,1 bis 5 Gew.-% des Gesamtgewichts der Zusammensetzung vorliegt/vorliegen.

11. Zusammensetzung nach Anspruch 10, in der die Verbindungen der Formel (I) in Salze übergeführt werden und der Formel (I') entsprechen: 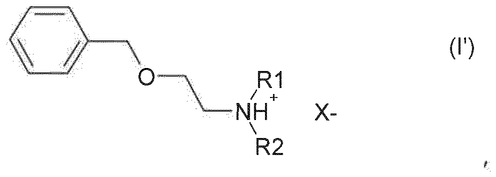 worin R1 und R2 unabhängig voneinander ein Wasserstoffatom oder einen Methyl-, Ethyl-, Phenyl- oder Benzylrest bedeuten; und X- ein kosmetisch unbedenkliches organisches und/oder anorganisches Anion oder eine Mischung von Anionen ist.

12. Zusammensetzung nach einem der Ansprüche 10 bis 11, in der die Verbindung der Formel (I) oder (I') aus den Folgenden ausgewählt ist:

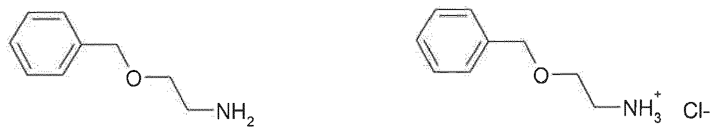

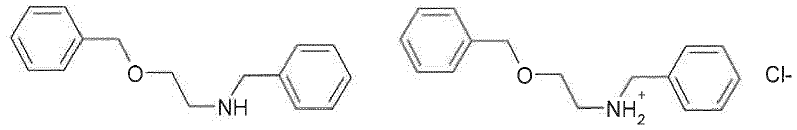

13. Zusammensetzung nach einem der Ansprüche 10-12, in der die Verbindung der Formel (I) und/oder eines ihrer Salze, allein oder in Mischung, in einer Menge zwischen 0,25 bis 3 Gew.-%, stärker bevorzugt 0,5 bis 2 Gew.-%, des Gesamtgewichts der Zusammensetzung vorliegt.

14. Zusammensetzung nach einem der Ansprüche 10 bis 13, in der das physiologisch unbedenkliche Medium mindestens einen Bestandteil umfasst, der aus den Folgenden ausgewählt ist: silikonhaltige Fettkörper wie Silikonöle, -gummen und -wachse; silikonfreie Fettkörper wie Öle, Pasten und Wachse pflanzlichen, mineralischen, tierischen und/oder synthetischen Ursprungs; ein wässriges Medium; ein wässrig-alkoholisches Medium, das einen einwertigen C₂-C₆-Alkohol enthält; C₂-C₆-Alkohole, Glykole wie Propylenglykol; Ketone; Emulgatoren, Tenside, Verdickungsmittel oder hydrophile oder lipophile Gelbildner, insbesondere kosmetische Wirkstoffe, Antioxidantien, Duftstoffe, Füllstoffe, Pigmente, UV-Filter, Geruchsabsorbenzien, Farbstoffe, feuchtigkeitsspendende Mittel, Vitamine, essenzielle Fettsäuren, Polymere, insbesondere lipidlösliche Polymere, Opakisierungsmittel, Stabilisatoren, Komplexbildner, Conditioner, Treibmittel.

15. Zusammensetzung nach einem der Ansprüche 10 bis 14, die in Form der Folgenden vorliegt:
- eines Make-up-Produkts der Haut des Gesichts, des Körpers oder der Lippen, wie einer Make-up-Grundlage, eines Rouge oder eines Lidschattens, eines Concealer-Sticks, eines Abdeck-Sticks, eines Eyeliners, einer Wimperntusche, eines Lippenstifts, eines Nagellacks, einer Nagelpflege;
- einer Sonnenschutzzusammensetzung oder einer Selbstbräunungszusammensetzung;
- eines Aftershave-Gels oder einer Aftershave-Lotion;
- einer Enthaarungscreme;
- einer Körperhygienezusammensetzung wie eines Deodorants, eines Duschgels oder eines Shampoos;
- einer pharmazeutischen Zusammensetzung;
- einer festen Zusammensetzung wie einer Seife oder eines Waschstücks;
- einer Zusammensetzung für Aerosol, die auch ein unter Druck stehendes Treibmittel umfasst;
- einer Lotion zum Wellenlegen, einer Frisiercreme oder eines Frisiergels, einer Färbezusammensetzung, insbesondere einer Oxidationszusammensetzung; einer restrukturierenden Lotion für das Haar, einer Dauerwellzusammensetzung, einer Lotion oder eines Gels gegen Haarausfall;
- einer Zusammensetzung für den Mund-Zahn-Bereich, zum Beispiel einer Zahncreme.

## Claims

1. Use of at least one compound of formula (I), or a salt thereof, as a preserving agent: 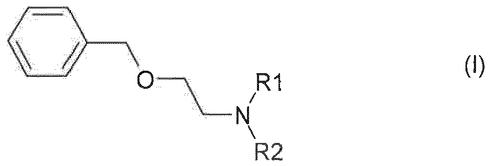 in which R1 and R2 represent, independently of one another, a hydrogen atom or a methyl, ethyl, phenyl or benzyl radical.

2. Use according to Claim 1, as a preserving agent of a cosmetic, dermatological or pharmaceutical composition.

3. Use according to either of the preceding claims, in which R1 represents H.

4. Use according to one of the preceding claims, in which R2 represents H or benzyl.

5. Use according to one of the preceding claims, in which the compounds of formula (I) are salified and correspond to the formula (I'): 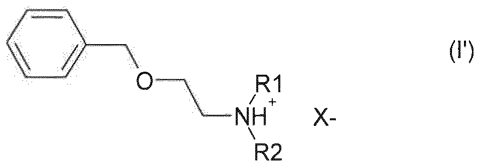 in which R1 and R2 are as defined in formula (I) and X-is an organic and/or mineral, cosmetically acceptable anion, or mixture of anions.

6. Use according to one of the preceding claims, in which the compound of formula (I) is chosen from:

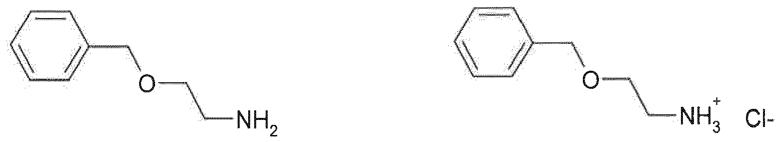

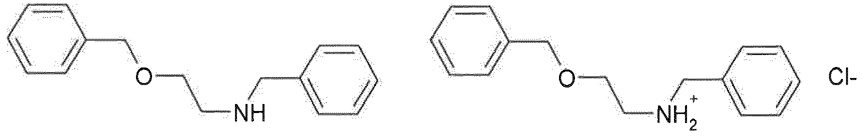

7. Use according to one of Claims 2 to 6, in which the compound of formula (I) and/or a salt thereof, alone or as a mixture, is present in the cosmetic, dermatological or pharmaceutical compositions in an amount between 0.1 and 5% by weight, preferably from 0.25 to 3% by weight, and more particularly 0.5 to 2% by weight, of the total weight of the composition.

8. Use according to one of Claims 2 to 6, in which the composition is in the form of:
- a product for making up the skin of the face, body or lips, such as a foundation, a face powder, an eye shadow, a concealer stick, a cover stick, an eyeliner, a mascara, a lipstick, a nail varnish or a nail care product;
- a sun protection composition or an artificial tanning composition;
- an aftershave gel or lotion;
- a hair-removing cream;
- a body hygiene composition such as a deodorant, a shower gel or a shampoo;
- a pharmaceutical composition;
- a solid composition such as a soap or a cleansing bar;
- an aerosol composition also comprising a pressurized propellent;
- a hairsetting lotion, a hair-styling cream or gel, a dye composition, in particular an oxidation dye composition, a hair-restructuring lotion, a permanent-wave composition, a lotion or a gel for combating hair loss; or
- a composition for buccodental use, for example a toothpaste.

9. Method for preserving a cosmetic, dermatological or pharmaceutical composition, **characterized in that** it consists in incorporating into said composition at least one compound of formula (I) or a salt thereof, as defined in any one of Claims 1 to 6.

10. Cosmetic, dermatological or pharmaceutical composition comprising, in a physiologically acceptable medium, at least one compound of formula (I), or a salt thereof: 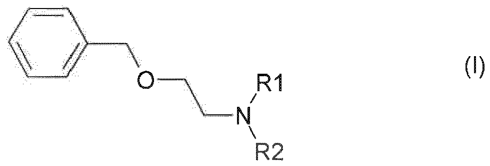 in which R1 and R2 represent, independently of one another, a hydrogen atom or a methyl, ethyl, phenyl or benzyl radical;
the compound of formula (I) and/or a salt thereof, alone or as a mixture, being present in an amount between 0.1 and 5% by weight, of the total weight of the composition.

11. Composition according to Claim 10, in which the compounds of formula (I) are salified and correspond to the formula (I') : 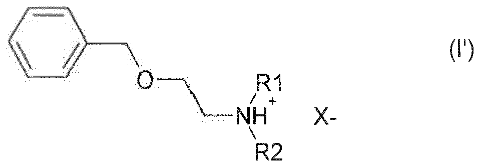 in which R1 and R2 represent, independently of one another, a hydrogen atom or a methyl, ethyl, phenyl or benzyl radical; and X- is an organic and/or mineral, cosmetically acceptable anion, or mixture of anions.

12. Composition according to either of Claims 10 and 11, in which the compound of formula (I) or (I') is chosen from:

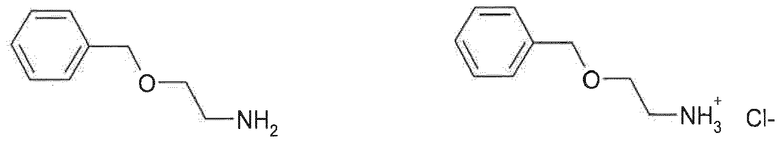

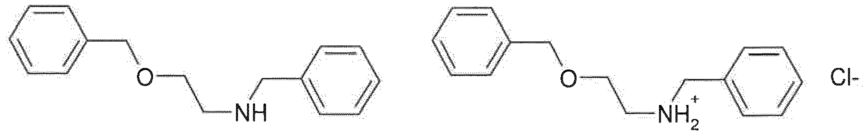

13. Composition according to one of Claims 10 to 12, in which the compound of formula (I) and/or a salt thereof, alone or as a mixture, is present in an amount between 0.25 and 3% by weight, and more particularly between 0.5 and 2% by weight, of the total weight of the composition.

14. Composition according to one of Claims 10 to 13, in which the physiologically acceptable medium comprises at least one ingredient chosen from: silicone fatty substances such as silicone oils, gums and waxes; non-silicone fatty substances such as oils, pastes and waxes of plant, mineral, animal and/or synthetic origin; an aqueous medium; an aqueous-alcoholic medium containing a C₂-C₆ monoalcohol; C₂-C₆ alcohols, glycols such as propylene glycol; ketones; emulsifiers, surfactants, hydrophilic or lypophilic thickeners or gelling agents, active agents, in particular cosmetic active agents, antioxidants, fragrances, fillers, pigments, UV-screening agents, odour absorbers, dyes, moisturizers, vitamins, essential fatty acids, polymers, especially liposoluble polymers, opacifiers, stabilizers, sequestrants, conditioners and propellants.

15. Composition according to one of Claims 10 to 14, which is in the form of:
- a product for making up the skin of the face, body or lips, such as a foundation, a face powder, an eye shadow, a concealer stick, a cover stick, an eyeliner, a mascara, a lipstick, a nail varnish or a nail care product;
- a sun protection composition or an artificial tanning composition;
- an aftershave gel or lotion;
- a hair-removing cream;
- a body hygiene composition such as a deodorant, a shower gel or a shampoo;
- a pharmaceutical composition;
- a solid composition such as a soap or a cleansing bar;
- an aerosol composition also comprising a pressurized propellent;
- a hairsetting lotion, a hair-styling cream or gel, a dye composition, in particular an oxidation dye composition, a hair-restructuring lotion, a permanent-wave composition, a lotion or a gel for combating hair loss; or
- a composition for buccodental use, for example a toothpaste.
